# FASCICULE DE BREVET EUROPEEN

(11) **EP 1 359 929 B1**
(45) Date de publication et mention de la délivrance du brevet: **13.07.2005**
(21) Numéro de dépôt: 01904035.1
(22) Date de dépôt: 02.02.2001
(51) Int. Cl.: A61K 35/84, A61P 39/00

(54) **COMPOSITION PHYTO-THERAPEUTIQUE PERMETTANT L'ELIMINATION DES METAUX LOURDS**
PHYTOTHERAPEUTISCHE ZUBEREITUNG ZUR ENTFERNUNG VON SCHWERMETALLEN
PHYTOTHERAPEUTIC COMPOSITION FOR ELIMINATING HEAVY METALS

(30) Priorité: 04.02.2000 FR 0001620
(43) Date de publication de la demande: 12.11.2003
(73) Titulaire: Mortal, Frédéric, 06000 Nice (FR)
(72) Inventeur: Mortal, Frédéric, 06000 Nice (FR)
(86) Numéro de dépôt international: PCT/FR2001/000318
(87) Numéro de publication internationale: WO 2001/056588

(56) Documents cités:
- DATABASE BIOSIS [en ligne] BIOSCIENCES INFORMATION SERVICE, PHILADELPHIA, PA, US; 1 novembre 1999 (1999-11-01) SCHIEWER SILKE ET AL: "Metal binding stoichiometry and isotherm choice in biosorption." Database accession no. PREV200000023237 XP002152454 & ENVIRONMENTAL SCIENCE & TECHNOLOGY, vol. 33, no. 21, 1 novembre 1999 (1999-11-01), pages 3821-3828, ISSN: 0013-936X

## Description

### Domaine technique

La présente invention concerne une composition nutritionnelle phyto-thérapeutique permettant l'élimination des métaux lourds contenus dans l'organisme.

### Etat de la technique

L'ère industrielle dans laquelle nous vivons, a des répercutions de plus en plus importantes sur l'environnement. En effet, la pollution de toute sorte fait aujourd'hui partie de notre quotidien. Parmi les polluants les plus pernicieux, les métaux lourds prennent une place de plus en plus importante. Les métaux que l'on retrouve dans l'organisme sont par exemple le plomb, le mercure ou le cadmium. Ces métaux sont source de toxicité qui se manifeste sous différentes formes : fatigue inexpliquée, troubles de l'humeur, troubles du sommeil, perturbations hormonales, maladies dégénératives, maladies cardio-vasculaires, maladies neurologiques.

Les restaurations métalliques dans les soins dentaires font que la bouche constitue une source importante de métaux. En effet, depuis des années, ces restaurations se font essentiellement par des « plombages » qui sont en fait des amalgames contenant 50 % de mercure, le reste étant de l'argent, de l'étain et du cuivre. D'autres sont à base d'or amalgamé à du palladium, du cuivre, de l'étain et de l'argent.

Dans ce milieu conducteur, les réactions chimiques sont nombreuses et notamment des réactions d'oxydation métallique. Les substances libérées peuvent être, par exemple, le mercure, le nickel, le cadmium ou encore l'aluminium.

L'effet toxique des métaux lourds est lié à leur grande affinité pour les groupes -SH qui se trouvent plus particulièrement au niveau des membranes cellulaires et notamment dans les protéines. La liaison d'un métal toxique avec un groupe -SH membranaire modifie la surface de la cellule. Une cellule ainsi modifiée peut devenir antigénique et se voir attaquée par les cellules du système immunitaire telles que les lymphocytes T, ce qui peut entraîner l'apparition de maladies auto-immunes. De même, ces métaux peuvent entraîner une neurotoxicité en agissant au niveau des cellules gliales du cerveau. Enfin, ils peuvent provoquer la formation de radicaux libres tels que les composés O⁻ et H₂O₂ qui sont à l'origine de la destruction des membranes.

On a montré que tous les individus n'étaient pas sensibles au mercure et qu'il fallait une prédisposition génétique pour développer les symptômes d'intoxication. Ainsi, il semble d'après les premières statistiques que 5 à 15 % de la population européenne est génétiquement sensible et réagit au mercure des amalgames dentaires, alors que 40 % de cette population réagit au nickel.

Une intoxication au mercure peut entraîner des tremblements qui peuvent conduire à une incapacité de travail. Les reins peuvent également être touchés par le biais de glomérulonéphrites.

Une intoxication au nickel a une action négative sur l'érythropoïèse puisqu'elle entraîne une diminution de la production d'hématies, ce qui déclenche des anémies sévères. De même, de trop fortes quantités de nickel entraînent un déséquilibre dans la population de leucocytes, puisque le nombre total de polynucléaires diminue alors que le nombre de polynucléaires éosinophiles, généralement très faible, augmente.

La plupart des thérapeutiques actuelles permettant l'élimination des métaux lourds sont employées dans un souci d'efficacité maximale. Aussi, elles sont extrêmement brutales au niveau des moyens qu'elles mettent en oeuvre et de la contrainte faite sur l'organisme. Ces produits évacuent indistinctement tous les métaux lourds y compris ceux qui sont utiles à l'organisme.

### Exposé de l'invention

Le but de l'invention est de pallier ces inconvénients en fournissant un produit nutritionnel à base d'éléments naturels qui traite en douceur les problèmes d'intoxication aux métaux lourds en permettant le drainage et l'élimination de ces derniers.

L'invention concerne donc une composition nutritionnelle phyto-thérapeutique, permettant l'élimination des métaux lourds de l'organisme. Cette composition comprend en particulier du Mycelium shitake, de l*'Auricularia auricula-judae*, du lichen, du saule blanc (*salix alba*), de l'ulve (*Ulva lactula*), de l'Acerola et des proanthocyanidol.

### Description détaillée de l'invention

La toxicité des métaux lourds est liée aux ponts indissolubles qui se forment dans l'organisme entre ces derniers et les différents types de protéines qu'il s'agisse d'enzymes ou de protéines de structure telles que celles qui forment les membranes cellulaires. Les enzymes se trouvent alors inactivées, et notamment les enzymes intervenant dans les défenses anti-radicalaires, entraînant ainsi une multitude d'anomalies au niveau du métabolisme.

Il est capital d'associer dans une synergie thérapeutique les processus de déchélation et de chélation. La déchélation est le processus physique qui consiste à couper les ponts indissolubles qui se sont créés entre les protéines et les métaux lourds. Ce processus permet de libérer les protéines des métaux. La chélation consiste à capter les métaux libérés et à les éliminer par les voies naturelles.

Le Mycelium shitake permet le passage de la paroi cellulaire. Il assure une action nutritive et revitalisante et casse les ponts métalliques existant au niveau des enzymes et des protéines. Il possède une importante activité immuno-stimulante.

L*'Auricularia auricula-judae* est un champignon. Il possède un fort pouvoir de captation des métaux lourds. Il a également des activités anti-inflammatoire et anti-tumorale très importantes.

Les proanthocyanidols (OPC) sont des substances végétales extraites du pépin de raisin aux propriétés anti-inflammatoires, anti-oxydantes et anti-radicalaires majeures. Elles possèdent une très bonne biodisponibilité. Ils agissent par effet capteur et stoppent les mécanismes de lésions multiples occasionnées par les radicaux libres, toujours en surproduction dans les métaux lourds. Ils ont une action détoxifiante hépatique et de protection vasculaire, dynamisant et stimulant la cinétique et l'élimination des métaux lourds.

L'ulve (*Ulva lactula*), appelée aussi « laitue des mers », est une algue verte lamelleuse très photophile, très riche en fibres (38 % du poids sec). Elle est également très riche en macro-éléments et en acides aminés indispensables. Elle pompe les métaux lourds et les élimine.

Les lichens du genre *Cetraria* sont riches en mucilage, principalement en lichénine (64 %), en isolichénine. Ils contiennent également d'autres composés tels que l'acide cétrarique, l'acide lichen stéarique ou l'acide usnique. Ainsi, on utilise préférentiellement le lichen d'Islande (*Cetraria Islanda*) et du lichen blanc (*Cetraria alba*).

L'Acérola est un fruit rouge de la famille des cerises, très riche en vitamines C ou acide ascorbique, composé qui a de remarquables propriétés réductrices et anti-radicalaires. Ce composé joue un rôle biochimique important en association avec des hydroxylases dépendantes des cytochromes P 450 microsomaux dans la transformation des substances toxiques. La vitamine C a des propriétés déchélatrices et chélatrices sur les ions métalliques en agissant sur leur adsorption, leur mobilisation et leur distribution.

Le saule blanc (*Salix alba*) contient des flavonoïdes (hétérosides), des tanins catéchiques (20 %) et des dérivés salicylés tels que le salicoside qui lui confèrent des propriétés anti-inflammatoires et déchélatrices.

La combinaison de ces végétaux, algues et lichens permet d'obtenir simultanément un effet déchélateur et un effet chélateur. Elle permet ainsi l'élimination des métaux lourds tout en respectant et en accentuant la physiologie émonctorielle et le métabolisme.

La composition comprend en outre de la maltodextrine et du stéarate de magnésium comme excipients.

Selon un mode de réalisation particulier, la composition est la suivante :

| | |
|---|---|
| Mycelium shitake | 20 mg |
| Auricularia auricula judae | 15 mg |
| Lichen d'Islande / *Cetraria islandila* | 70 mg |
| Lichen blanc / *Cetraria* | 50 mg |
| Saule / *Salix alba* | 55 mg |
| Ulve / *Ulva lactula* | 55 mg |
| Acérola (cerise) | 50 mg |
| Proanthocyanidols (OPC) | 25 mg |
| Maltodextrine | 52 mg |
| Stéarate de magnésium | 8 mg |

La présente composition se présente préférentiellement sous forme pulvérulente contenue dans des gélules à avaler.

Des tests ont été réalisés chez des patients victimes d'intoxication aux métaux lourds. Ces patients ont subi un traitement avec la composition selon l'invention. Ce traitement consistait à prendre trois gélules contenant la composition, trois fois par jour. Ce traitement a duré six mois.

Une première série de tests a consisté à doser les métaux lourds dans la salive et les cheveux.

Un dosage dans les urines a également été réalisé afin d'évaluer le taux d'évacuation des métaux lourds par les voies urinaires.

Le dosage dans les cheveux a permis d'évaluer la présence d'éléments à l'état de traces témoignant du contact avec des métaux lourds. L'analyse des cheveux s'effectue au spectromètre d'émission à plasma et porte essentiellement sur les rapports calcium / magnésium, cuivre / fer, manganèse / chrome et zinc / cuivre. Ces éléments sont physiologiquement équilibrés entre eux. Les perturbations de ces éléments épurateurs tels que le calcium, le zinc, le sélénium sont le reflet des intoxications aux métaux lourds, en particulier les rapports calcium / magnésium et zinc / cuivre. Ces principaux oligo-minéraux jouent un rôle important en tant que facteurs enzymatiques en contrôlant la régulation d'activités enzymatiques. Ils interviennent également dans le transport et le métabolisme de l'oxygène.

Dans les interactions entre métaux, les ions cadmium (Cd²⁺) et mercure (Hg²⁺) possèdent la même structure électronique (couche externe) que les ions Cuivre (Cu²⁺) et zinc (Zn²⁺) et peuvent ainsi les remplacer.

Concernant le mercure, Les résultats des analyses ont permis de montrer que le taux d'évacuation quotidienne a augmenté entre 50 % et 120 %. Dans le cas de teneurs très importantes en mercure, on constate que le cuivre et le zinc sont éliminés en premier dans le processus de déchélation. Cette élimination primaire préalable à celle du mercure dans un premier temps, va diminuer dans un deuxième au profit de l'élimination du mercure qui sera alors plus importante. L'élimination du mercure dans les urines, se traduit par une diminution significative des traces de mercure dans les cheveux à partir de la cinquième ou sixième semaine de traitement. Cette diminution va de 15 % dans les cas les moins favorables à 40 % dans les cas les plus favorables.

En ce qui concerne le cadmium, métal très toxique, il est éliminé en priorité dans le processus de travail de la composition. On observe une disparition totale de ce métal après un traitement minimum de quinze jours et un traitement moyen d'un mois.

Des tests sanguins ont également été réalisés sur la même population. Ces tests ont pour but de mettre en évidence le niveau de stimulation des lymphocytes T. Le test employé est le test MELISA (Memory Lymphocyte Immuno-Stimulation Assay). Ce test permet de mesurer avec 100 % de fiabilité, la susceptibilité des patients aux métaux lourds, en déterminant si les lymphocytes T d'un patient possèdent des récepteurs membranaires pour des métaux lourds couplés à des structures organiques. En effet, un métal n'est pas antigénique par lui-même, il doit être couplé à un transporteur dont il a modifié la structure.

L'indice de stimulation des lymphocytes T a été mesuré au début du traitement avec la composition selon l'invention, trois mois et six mois après le début du traitement.

En ce qui concerne l'intoxication à l'or, les patients qui avaient un indice de stimulation supérieure à la normale ont vu cet indice diminuer dès le troisième mois et revenir à la normale au bout du sixième mois de traitement et ceci dans 100 % des cas.

En ce qui concerne l'intoxication au nickel, les patients qui avaient un indice de stimulation élevé au début du traitement ont vu celui-ci augmenter, dans la plupart des cas, au troisième mois. Au sixième mois, seul un patient devait continuer le traitement, l'indice des autres patients étant revenu à la normale.

En ce qui concerne l'intoxication au mercure, 50 % des patients qui avaient un indice supérieur à la normale ont vu celui-ci retrouver une valeur normale au bout de trois mois de traitement. Parmi les autres patients, un seul a dû poursuivre le traitement au-delà de six mois, son indice n'étant pas revenu à la normale.

En ce qui concerne l'intoxication au cadmium, deux tiers des patients ont vu leur indice augmenter à trois mois. A six mois, seul un patient a dû poursuivre son traitement.

Ces tests ont été complétés par un questionnaire rempli par les patients concernant les symptômes auxquels ils étaient sujet. Un tableau récapitulant les symptômes et leur fréquence avant traitement est présenté ci-après :

| **Symptômes** | **Fréquence chez les patients** |
|---|---|
| -Agitation, nervosité -Voies respiratoires, toux | 60 % |
| -Problèmes de vision, troubles oculaires -Troubles gastro-intestinaux -Manque de dynamisme -Chute de cheveux | 50 % |
| -Maux de tête, migraine -Goût métallique -Dépression nerveuse | 40 % |
| -Eczéma, éruption cutanée -Bourdonnement d'oreilles -Troubles circulatoires, vertiges -Palpitations cardiaques -Rhume des foins -Rhumatisme, douleurs articulaires -Spasmophilie -Perte d'appétit -Gorge, refroidissement | 33 % |
| -Faiblesse de concentration mentale -Asthme -Perte d'appétit | 23 % |

Parmi ces symptômes, certains ont disparu chez tous les patients après un traitement de six mois avec la composition selon l'invention :
- Problèmes de vision, troubles oculaires
- Chute de cheveux
- Maux de tête, migraine
- Goût métallique
- Dépression nerveuse
- Eczéma, éruption cutanée
- Bourdonnement d'oreilles
- Perte d'appétit
- Faiblesse de concentration mentale
- Asthme

D'autres symptômes n'ont disparu chez deux tiers des patients :
- Agitation, nervosité
- Voies respiratoires, toux
- Troubles gastro-intestinaux
- Manque de dynamisme
- Troubles circulatoires, vertiges
- Palpitations cardiaques
- Rhume des foins
- Rhumatisme, douleurs articulaires
- Spasmophilie
- Gorge, refroidissement

La composition selon l'invention permet donc de diminuer de façon très significative l'indice de stimulation des lymphocytes T face aux métaux lourds tels que le mercure, le nickel, le cadmium, l'aluminium et l'or. Cet indice atteint en fin de traitement une valeur nettement en dessous du seuil d'intolérance aux métaux lourds.

Cette composition permet également la disparition d'un grand nombre de symptômes dont sont victimes les patients.

La composition selon l'invention représente donc un moyen efficace pour lutter contre l'intoxication aux métaux, notamment chez les patients qui ont subi des soins dentaires avec l'utilisation d'amalgames contenant ces métaux. En cela, la présente composition représente un outil particulièrement intéressant pour les dentistes qui souhaitent prescrire un traitement à leur patient après une intervention, afin de leur éviter tout risque d'intoxication ou d'intolérance.

## Revendications

1. Composition nutritionnelle phyto-thérapeutique permettant l'élimination des métaux lourds de l'organisme **caractérisée en ce qu'**elle comprend en particulier du Mycelium shitake, de l'*Auricularia auricula-judae*, du lichen, du saule blanc (*Salix alba*), de l'ulve *(Ulva lactula*), de l'Acérola et des proanthocyanidols (OPC).

2. Composition selon la revendication 1, dans laquelle le lichen appartient au genre *Cetraria*.

3. Composition selon la revendication 2, dans laquelle le lichen est du lichen d'Islande (*Cetraria Islandila*) et du lichen blanc (*Cetraria alba*).

4. Composition selon l'une des revendications précédentes **caractérisée en ce qu'**elle comprend également de la maltodextrine et du stéarate de magnésium comme excipients.

5. Composition selon la revendication 4, dans laquelle la quantité de maltodextrine est comprise entre 30 et 70 mg.

6. Composition selon l'une des revendications 4 ou 5, dans laquelle la quantité de stéarate de magnésium est comprise entre 5 et 10 mg.

7. Composition selon la revendication 3, dans laquelle la quantité de Mycelium shitake est comprise entre 5 et 35 mg.

8. Composition selon la revendication 3, dans laquelle la quantité d'*Auricularia auricula judae* est comprise entre 5 et 30 mg.

9. Composition selon la revendication 3, dans laquelle la quantité de lichen d'Islande est comprise entre 40 et 120 mg.

10. Composition selon la revendication 3, dans laquelle la quantité de lichen blanc est comprise entre 40 et 90 mg.

11. Composition selon l'une des revendications précédentes, dans laquelle la quantité de saule est comprise entre 30 et 90 mg.

12. Composition selon l'une des revendications précédentes, dans laquelle la quantité d'ulve est comprise entre 30 et 90 mg.

13. Composition selon l'une des revendications précédentes, dans laquelle la quantité d'Acerola est comprise entre 25 et 75 mg.

14. Composition selon l'une des revendications précédentes, dans laquelle la quantité de proanthocyanidols est comprise entre 10 et 60 mg.

15. Composition selon l'une des revendications précédentes, utilisée dans un médicament pour traiter des patients ayant subi des soins dentaires avec utilisation d'amalgames, afin de leur éviter une intoxication aux métaux lourds.

## Claims

1. The nutntional composition phyto-therapeuric allows the elimination of heavy metals in the organism **characterized by** its content in particular of Mycelium shitake, Auncularia aucirula-judae, lichen, white willow(Salix alba), ulve (ulva lactula), Acerola and of proanthocyanidols (OPC)

2. Composition such as claimed in 1, in that lichen belongs to the Cetraria type.

3. Composition such as claimed in 2, in that lichen is of Island lichen (Cetraria Islandila) and of white lichen (Cetrana alba).

4. Composition from one of the claims earlier **characterized in that** it contains also maltodextrine and magnesium stearate as excipients.

5. Composition from claim 4, in that the quantity of maltodextrine comprised between 30 and 70 mg.

6. Composition from one of the claim 4 or 5, in that the quantity of magnesium stearate is comprised of 5 to 10 mg.

7. Composition from claim 3, in that the quantity of Mycelium shitake is comprise between 5 and 35 mg.

8. Composition from claim 3, in that the quantity of Auricularia auricular judae is comprised between 5 and 30 mg.

9. Composition from claim 3, in that the quantity of Island lichen is comprised between 40 and 120 mg.

10. Composition from claim 3, in that the quantity of white lichen is comprised between 40 and 90 mg.

11. Composition from one of the earlier claims, in that the quantity of willow is comprised between 30 and 90 mg.

12. Composition from one of the earlier claims, in that the quantity of ulve is comprised between 30 and 90 mg.

13. Composition from one of the earlier claims, in that the quantity of Acerola is comprised between 25 and 75 mg.

14. Composition from one of the earlier claims, in that the quantity of proanthocyanidols is comprised between 10 and 60 mg

15. Composition from one of the earlier claims, used in a medication for treatment of patients being subjected to dental care that utilized amalgams in order to prevent intoxication to heavy metals.

## Patentansprüche

1. Pflanzentherapeutische Nahrungszusammensetzung, die die Beseitigung der Schwermetalle aus dem Organismus ermöglicht, kennzeichnet sich **dadurch**, dass sie besonders aus "Mycelium shitaki", "auricularia auricula-judae" Flechte, Pappelweide (salix alba), Ulve (Ulva Lactula), aus Acerola und proanthocyanidols (OPC) besteht.

2. Zusammensetzung nach der Forderung 1, in welchem die Flechte der Gattung "cetrana" angehört.

3. Zusammensetzung nach der Forderung 2, in welcher die Flechte, Flechte aus Island (cetrana islandila) und weiße Flechte (cetrana alba) ist.

4. Zusammensetzung nach einer Forderung, die sich **dadurch** charakterisiert, dass sie als Bindemittel "maltodextrine" und "stearate de magnesiujm" benutzt.

5. Zusammensetzung nach der Forderung 4, in welcher die Quantität der "maltodextrine" zwischen 30 und 70 mg beträgt.

6. Zusammensetzung nach einer Forderung 4 oder 5, in welcher die Quantität der "stearate de magnesium" zwischen 5 und 10 mg beträgt.

7. Zusammensetzung nach der Forderung 3, in welcher die Quantität von "Mycelium shitake" zwischen 5 und 35 mg beträgt.

8. Zusammensetzung nach der Forderung 3, nach welcher die Menge von "auricula judae" zwischen 5 und 30 mg beträgt

9. Zusammensetzung nach der Forderung 3, nach welcher die Quantität der Flechte aus Island zwischen 40 und 12o mg beträgt.

10. Zusammensetzung nach der Forderung 3, nach welcher die Quantität der weißen Flechte zwischen 40 und 90 mg beträgt.

11. Zusammensetzung nach einer Forderung der vorherigen Forderungen, nach welcher die Quantität der Weide zwischen 30 und 90 mg beträgt.

12. Zusammensetzung nach einer der vorherigen Forderungen, nach welcher die Quantität von Ulve zwischen 30 und 90 mg beträgt.

13. Zusammensetzung nach einer der vorherigen Forderungen, nach welcher die Quantität von "acerola" zwischen 25 und 75 mg beträgt.

14. Zusammensetzung nach einer der vorherigen Forderungen, nach welcher die Quantität von "proanthocyanidols" zwischen 10 und 60 mg beträgt.

15. Zusammensetzung nach einer der vorherigen Forderungen, die als Medikament benutzt wird, um Patienten zu behandeln, die sich einer Zahnpflege mit Amalgam unterzogen haben, damit eine Vergiftung mit den Schwermetallen vermieden wird.
